(19) Europäisches Patentamt
European Patent Office
Office européen des brevets

(11) Veröffentlichungsnummer: **0 008 682**
**A1**

(12) # EUROPÄISCHE PATENTANMELDUNG

(21) Anmeldenummer: 79102770.9

(22) Anmeldetag: 02.08.79

(51) Int. Cl.³: G 01 N 33/54
C 07 G 7/00, C 08 F 8/00

(30) Priorität: 02.08.78 GB 3192978

(43) Veröffentlichungstag der Anmeldung:
19.03.80 Patentblatt 80/6

(84) Benannte Vertragsstaaten:
BE CH DE FR GB IT NL SE

(71) Anmelder: F. Hoffmann-La Roche & Co.
Aktiengesellschaft
Abt. RPM/P
CH-4002 Basel(CH)

(72) Erfinder: Fischer, Ernst Alfred Dr.
Rüttihardstrasse 2
CH-4142 Münchenstein(CH)

(74) Vertreter: Lederer, Franz, Dr. et al,
Patentanwälte Lederer, Meyer Lucile-Grahn-Strasse 22
D-8000 München 80(DE)

(54) **Mit einem Proteinmaterial beschichteter Latex, Verfahren zur Herstellung dieses Latex, immunologisches Reagenz enthaltend diesen Latex, Verfahren zur Herstellung dieses Reagenzes, Verwendung dieses Reagenzes, Bestimmungsverfahren unter Verwendung dieses Reagenzes und Reagenziengarnitur enthaltend dieses Reagenz.**

(57) Es wird beschrieben ein Latex mit diskreten Teilchen eines Latex-Trägers, an den ein Proteinmaterial kovalent gebunden ist; ein Verfahren zur Herstellung dieses Latex; ein immunologisches Reagenz bestehend aus diesem Latex beschichtet mit einem immunologisch aktiven Material; ein Verfahren zur Herstellung dieses Reagenzes; die Verwendung dieses Reagenzes; ein Bestimmungsverfahren unter Verwendung dieses Reagenzes und eine Reagenziengarnitur enthaltend dieses Reagenz.

EP 0 008 682 A1

Croydon Printing Company Ltd.

2. Aug. 1979

F. Hoffmann-La Roche & Co. Aktiengesellschaft, Basel, Schweiz 0008682

Vorklassifizierungsnummer: C.P.79/001680 RAN 4093/43

Mit einem Proteinmaterial beschichteter Latex, Verfahren zur Herstellung dieses Latex, immunologisches Reagenz enthaltend diesen Latex, Verfahren zur Herstellung dieses Reagenzes, Verwendung dieses Reagenzes, Bestimmungsverfahren unter Verwendung dieses Reagenzes und Reagenziengarnitur enthaltend dieses Reagenz.

Die vorliegende Erfindung betrifft ein neues, aktiviertes Latex-Polymer sowie ein Verfahren zu dessen Herstellung. Sie bezieht sich ferner auf diagnostisch brauchbare Reagenzien, ein Verfahren zu deren Herstellung und solche Reagenzien verwendende Diagnosemethoden.

Die Diagnose pathologischer Zustände oder anderer Umstände im Menschen sowie bei Tieren erfolgt zunehmend unter Anwendung immunologischer Prinzipien, die zur Bestimmung des Vorhandenseins von Antikörpern oder Antigenen in einer Körperflüssigkeit des Versuchsobjekts angewandt werden.

Unter den verschiedenen bekannten Testmethoden, wie dem Agglutinationstest, dem Radioimmunoassay, dem Enzymimmunoassay, der Immunofluoreszenz oder der Immunodiffusion wird der Agglutinationstest als der billigste, einfachste und schnellste angesehen.

Bei einem Agglutinationstest werden Träger verwendet, um die visuelle oder photometrische Unterscheidung der gebildeten Antigen-Antikörper-Komplexe, die von sehr geringer Grösse sind, zu ermöglichen. Unter den Trägern, die verwendet worden sind, sind Schaf- und Human-Erythrozyten, Bakterienzellen, Bentonit, Latex-Teilchen, z.B. Polystyrol, oder carboxylierte Copolymerisate aus Styrol und Butadien, anionische Phenolharze

Klt/11.7.1979

und fein zerteilte diazotierte Aminocellulose.

Die bekannten Träger sind jedoch in ihrer Anwendbarkeit und Brauchbarkeit bei immunologisch-diagnostischen Arbeitsweisen beschränkt, da die entsprechenden immunologischen Reagenzien in vielen Fällen mangelnde Empfindlichkeit und/oder geringe Stabilität aufweisen. Dies ist insbesondere dann der Fall, wenn die immunologischen Reagenzien empfindliche Antigene oder Antikörper umfassen, die an den Träger gebunden sind.

Es war insbesondere nicht möglich, einen Agglutinationstest zur Bestimmung von Myoglobin im Urin von Patienten mit Verdacht auf Myokardinfarkt zu entwickeln, weil die bekannten immunologischen Träger beim Befestigen von Myoglobin unter Erhalt seiner immunologischen Eigenschaften nicht befriedigen oder nicht genügend Stabilität zeigen. Die bekannten Verfahren zur Bestimmung von Myoglobin im Urin, wie die Radioimmunodiffusion, sind teuer und mühselig. Insbesondere erlauben sie keine rasche Abschätzung des Myoglobins im Urin, die der Arzt am Bettrand des Patienten mit Verdacht auf Myokardininfarkt vornehmen könnte.

Es besteht also ein Bedürfnis nach einem Träger, der mit einem breiten Spektrum immunologisch aktiver Materialien, insbesondere mit Myoglobin, ein diagnostisch brauchbares Reagens bildet, das stabil, spezifisch, empfindlich ist und eine leicht festzustellende visuelle oder photometrische Auswertung in kürzester Zeit zulässt.

Die Erfindung bezieht sich auf einen aktivierten Latex, der durch Behandeln diskreter Teilchen eines Latex-Trägers hergestellt wird, an den ein wasserlösliches Proteinmaterial kovalent mit einem Aktivierungsmittel gebunden ist.

Die Erfindung bezieht sich weiter auf ein wasserunlösliches immunologisches Reagens mit einem spezifischen Gewicht von etwa dem von Wasser, das diskrete Teilchen eines Latex-Trägers aufweist, an den ein wasserlösliches Proteinmaterial kovalent gebunden ist, woran über eine kovalente Bindung ein bekanntes immunologisch aktives Material kondensiert ist.

Schliesslich bezieht sich die Erfindung auf ein Verfahren zur Herstellung eines solchen wasserunlöslichen immunologischen Reagens, wobei ein immunologisch aktives Material mit einem aktivierten Latex, hergestellt durch Behandeln diskreter Teilchen eines Latex-Trägers, an den ein wasserlösliches Proteinmaterial kovalent gebunden ist, mit einem Aktivierungsmittel, umgesetzt wird.

Antigene oder Antikörper, welche erfindungsgemäss an proteinüberzogene Latex-Kügelchen gebunden sind, befinden sich in sehr natürlicher Umgebung und zeigen hohe biologische Aktivität und haben günstige Eigenschaften hinsichtlich der bei normalem menschlichem Serum beobachteten Latex-Agglutination.

Der im Zusammenhang mit der Erfindung gebrauchte Ausdruck "Latex-Träger" oder "Kernpolymer" umfasst Latex-Polymere, die wasserunlöslich sind, eine Teilchengrösse im Bereich von etwa 0,01 bis etwa 0,9 µm und ein spezifisches Gewicht nahe dem von Wasser haben, so dass nach dem Ueberziehen mit einem Proteinmaterial und Kuppeln mit dem immunologisch aktiven Material das spezifische Gewicht der Teilchen etwa 1,0 (0,95-1,05) beträgt, was sie in die Lage versetzt, ständig in wässriger Suspension zu verbleiben. Das Kernpolymer muss im Hinblick auf immunologisch-diagnostische Tests inert sein und aktive Gruppen aufweisen, die eine kovalente

Bindung mit einem Proteinmaterial auszubilden vermögen. Beispielsweise können die Latex-Träger Carboxylgruppen, Amingruppen oder in diese überführbare Gruppen haben. Typische geeignete Gruppen an den Latex-Trägern sind solche, die einen aktiven Wasserstoff enthalten, z.B. -COOH, $-CONH_2$, oder eine Nitrilgruppe, eine primäre Amingruppe oder eine sekundäre Amingruppe.

Typische geeignete Latex-Teilchen sind solche, die als wässrige Latex-Suspension im Handel sind, gewöhnlich in Konzentrationen von etwa 40 bis etwa 60 o/o Feststoffen. Viele Arten Latex-Polymere sind zur erfindungsgemässen Verwendung geeignet, vorausgesetzt sie erfüllen die oben angeführten Kriterien. Die Erfindung umfasst die Verwendung all dieser geeigneten Latices.

Bevorzugte Latexpolymere sind carboxylierte Latex-Polymere, wie carboxylierte Styrol-Butadiene, carboxylierte Polystyrole, carboxylierte Polystyrole mit Aminogruppen, Acrylsäure-Polymere, Metharcylsäure-Polymere, Acrylnitril-Polymere, Acrylnitril-Butadien-Styrole, Polyvinylacetat-Acrylate, Polyvinylpyridine, Vinylchlorid-Acrylate und dergleichen. Einige im Handel erhältliche Latices, die zur erfindungsgemässen Verwendung geeignet sind, sind Amsco Res 4150, Amsco Res 3011 (American Mineral Spirits Co.); Dow Latex 815, Dow Latex 816, Dow Latex 620, Dow Latex 859, Dow Latex CL 241 (The Dow Chemical Co.); Hycar 1512, Hycar 1877X8, Hycar 2600x120 (Goodrich Chemical Co.); Gelva 900, Lytron 612, Lytron 624 (Monsanto); Rhoplex LC 3216, Amberlite Ultrafine (Röhm und Haas).

Besonders bevorzugte Latex-Polymere sind carboxylierte Styrol-Butadiene.

Der Begriff Protein-Material umfasst alle Aminosäure-Polymere , welche für die kovalente Bindung von immunologisch aktiven Materialien geeignet sind. Bevorzugte Protein-Materialien sind wasserlösliche Proteine, Polypeptide oder Peptide. Beispiele solcher Protein-Materialien sind Albumine und Globuline. Besonders bevorzugt sind Rinderserumalbumin und Rinder-Gammaglobulin.

Der Ausdruck "immunologisch aktives Material" bezieht sich auf Komponenten physiologischer Flüssigkeiten, Zell- und Gewebeextrakte, wofür ein immunologisches Gegenreagens zur Verfügung steht oder erzeugt werden kann. Typische immunologische Materialien sind primäre Amine, Aminosäuren, Peptide, Proteine, Lipoproteine, Glykoproteine, Sterine, Steroide, Lipoide, Nukleinsäuren, Enzyme, Hormone, Vitamine, Polysaccharide und Alkaloide.

Beispiele für solche immunologisch aktive Substanzen sind in der folgenden Tabelle aufgeführt:

Tabelle I

I. Mikroorganismen

Bakterien

1. Gram-positive Kokken
   Streptokokken (pyogenes, fecalis und viridans)
   Staphylokokken (aureus und albus)
   Pneumokokken (D. pneumoniae)
2. Gram-negative Kokken
   Neisseria (gonorrhoeae und meningitidis)
3. Gram-positive aerobe Bazillen
   Bacillus anthracis
   Corynebacterium diphtheriae
   Erysipelothrix
   Listeria monocytogenes

4. Gram-positive anaerobe Bazillen
   Clostridia (botulinum, perfringens, welchii und tetani)
5. Gram-negative anaerobe Bazillen
   Bacteroides
6. Gram-negative Intestinum-Bazillen
   Escherichia
   Klebsiella
   Enterobacter
   Proteus
   Pseudomonas
   Salmonella
   Shigella
7. Gram-negative nicht-intestinale Bazillen
   Pasteurella (pestis und tularensis)
   Hemophilus influenzae
   Brucella (meltitensis, abortus und suis)
   Bordetella pertussis
   Malleomyces
8. Spirochäten
   Treponema pallidum
   Leptospira
   Borrelia
9. Mycoplasma
10. Mycobacteria
11. Vibrio
12. Actinomyces

<u>Protozoen</u>

1. Intestinale Protozoen
   Amöben
2. Flagellaten
   Trichomonas
   Leishmania
   Trypanosomen
   Toxoplasma

3. Sporozoen
   Plasmodien (vivax, falciparum, malariae und ovale)
4. Intestinale Nematoden
   Maden- oder Springwürmer
   Hakenwürmer
   Peitschenwürmer
5. Gewebe-Nematoden
   Trichinella
   Filaria (Wuchereria bancroftii)
   Dracunculus
6. Trematoden
   Schistosomen
   Eingeweide-Saugwürmer
   Gewebe-Egel
7. Cestoden
   Bandwürmer
8. Toxoplasma (T. gondii)

Fungi

1. Sporotrichum
2. Cryptococcus
3. Blastomyces
4. Histoplasma
5. Coccidioides
6. Candida

Viren und Rickettsien

1. Rickettsien
2. Viren
   Hunde-Hepatitis
   Shope-Papillome
   Influenza A + B
   Hühnerpest
   Herpes simplex
   Adenoviren
   Polyome

Rous' Sarkom
Impfpocken
Poliovirus
Masern
Hundestaupe
Leukämie
Mumps
Newcastle-Krankheit
Sendai
ECHO
Maul- und Klauenseuche
Psittacosis
Tollwut
Extromelia
Baumviren

II. Gewebe-Antigene, einschliesslich organspezifische Antigene

Polysaccharide
Hyaluronidase
Tetanus-Toxin
Ei-Ovalbumin
Ei-Serumalbumin
Niere
Leber
Haut
Herz (Myoglobin)
Magen-Darm-Trakt
Prostata
Embryo-Antigene (alpha 1 Fetoprotein)
Tumor-Antigene (Carcinoembryo-Antigen)
Muskel
Kollagen
Amyloid

III. Hormone

Hypophysen-Hormone
Insulin
Glucagon
Thyroid-Hormon
Choriongonadotropin
choriones Wachstumshormon - Prolactin
Human-Placenta-Lactogen

IV. Enzyme

Pankreas-Chymotrypsinogen
Procarboxypeptidase
Desoxyribonuclease
Ribonuclease
Glyceraldehyd-3-phosphat-dehydrogenase
Katalase
Peroxidase

V. Blutzellen-Antigene, Blutgruppensubstanzen und andere Isoantigene

Blutplättchen
Megakaryozyten
Leukozyten
Erythrozyten
Blutgruppensubstanzen
Forssmann-Antigen
Histokompatibilitäts-Antigene

VI. Plasma-Proteine

Fibrin und Fibrinogen
Plasminogen und Plasmin
Albumin
Immunoglobuline

α-1-Antichymotrypsin
α-1-Antitrypsin
Komplement-Faktoren
Ceruloplasmin
Gc-Globulin
Haptoglobin
α-2-Makroglobulin
ß-2-Mikroglobulin
Orosomucoid
Prealbumin
Transferrin

VII. <u>Milch-Proteine</u>

Lactoferrin
Lysozym
Sekret-IgA
Sekret-IgM
Sekret-Komponente

VIII. <u>Speichel-Proteine</u>

Sekret-IgA
Sekret-IgM
Sekret-Komponente

IX. Urin-Proteine

X. <u>Pathologische Proteine</u>

Myeloma-Protein
Makroglobulinaemische Proteine
Dysglobulinaemische Proteine
Bence Jones I, II-Proteine
C-reaktives Protein
Kryoglobuline

XI. Antikörper, einschliesslich Autoantikörper

Antikernfaktor
Thyroid-Autoantikörper
Anti-Tamm-Horsfall-Protein
Kalt-Agglutinine
Rheumatoid-Faktor
Adrenal-Autoantikörper
Autoantikörper zu Magenwandzellen bei perniziöser Anämie
Anti-Colon
Anti-Leber
Anti-Niere
Autoantikörper zu Spermatozoen
Anti-Herz
Muskel-Autoantikörper bei Myasthenia gravis
Autoantikörper zu Nervengewebe
Autoantikörper gegen Fasergewebe und Gefässkomponenten
Autoantikörper gegen Blutplättchen und Megakaryozyten
Antikörper gegen Trophoblasten
Antikörper zu Mikroorganismen
Antikörper zu tierischen Antigenen
Antikörper zu Arzneimitteln

Ein besonders bevorzugtes immunologisch aktives Material ist Myoglobin, Human-Placenta—Lactogen, Antikörper gegen IgG oder Streptolysin O.

Der neue aktivierte Latex gemäss vorliegender Erfindung kann durch

a) Reaktion einer wässrigen Latex-Suspension mit einem Protein-Material und

b) Reaktion des erhaltenen Produktes mit einem Aktivierungsmittel

hergestellt werden.

Stufe a) kann in herkömmlicher Weise durchgeführt werden; allerdings in Abhängigkeit von den aktiven funktionellen Gruppen des verwendeten Latex sowie der Art des verwendeten Protein-Materials.

In einer bevorzugten Ausführungsweise, bei der der verwendete Latex ein carboxyliertes Polymer ist, wird Stufe a), bei der eine Amidbindung ausgebildet wird, in Gegenwart eines Kupplungsmittels, wie eines wasserlöslichen Carbodiimids, des Woodward-Reagens K (N-Aethyl-5-phenyl-isoxazolium-3'-sulfonat) oder eines wasserlöslichen Chlorformiats durchgeführt.

Besonders bevorzugte Kupplungsmittel sind wasserlösliche Carbodiimide der Formel

$$R-N=C=N-R' \qquad I$$

worin R oder R' Cycloalkyl mit 5 bis 6 Kohlenstoffatomen im Ring, Alkyl mit 2 bis 12 Kohlenstoffatomen, z.B. Aethyl, n-Propyl, Isopropyl, n-Butyl, sec.-Butyl, Isobutyl, tert.-Butyl, Amyl, Hexyl, Heptyl, Octyl, Nonyl, Undecyl und Dodecyl, monoarylsubstituierte Niederalkylreste, z.B. Benzyl, α- und ß-Phenyläthyl, Monoarylreste, z.B. Phenyl, Morpholino, Piperidyl, morpholinylsubstituierte Niederalkylreste, z.B. Aethylmorpholinyl, piperidylsubstituierte Niederalkylreste, z.B. Aethylpiperidyl, Diniederalkylamino-Niederalkylreste, pyridylsubstituierte Niederalkylreste, z.B. α-, ß- und γ-Methyl- oder -Aethylpyridyl sind,

deren Säureadditionssalze und quaternäre Ammoniumsalze.

Nach einer bevorzugten Ausführungsform der Erfindung wird in Stufe a) das Protein-Material und eine wässrige Suspension des carboxylierten Kernlatex vorzugsweise bei Raumtemperatur (etwa 20 bis etwa 25°C) umgesetzt. Temperaturen von etwa 0 bis etwa 40°C sind jedoch für die Umsetzung auch geeignet. Um ein chemisches Kuppeln des Protein-Materials an den Kernlatex zu gewährleisten, wird eine ausreichende Menge an Kupplungsmittel verwendet, um sicherzustellen, dass genügend Amidbindungen ausgebildet werden. Im allgemeinen sind etwa 0,005 bis etwa 6,0 Gewichtsprozent an wasserlöslichem Carbodiimid, bezogen auf das Gewicht der Teilchen, geeignet, gewöhnlich werden jedoch etwa 0,05 bis 2,0 Gewichtsprozent verwendet.

Der pH der Reaktion kann zwischen 2 und 7 variieren und liegt vorzugsweise bei 4 bis 5.

Die Reihenfolge der Zugabe des Proteinmaterials, des carboxylierten Kernlatex und des Kupplungsmittels ist unkritisch. Vorzugsweise wird jedoch in einer ersten Stufe das Kernlatex-Polymer mit dem wasserlöslichen Kupplungsmittel umgesetzt und in einer zweiten Stufe das Protein-Material zugefügt.

Das Verhältnis von Kernlatex zu Protein-Material in dem anfallenden Produkt kann innerhalb breiter Grenzen variieren und liegt vorzugsweise zwischen 2 und 150, wobei ein Verhältnis von 10 bis 50 besonders bevorzugt wird.

In Stufe b) des Verfahrens gemäss vorliegender Erfindung wird der Latex gemäss Stufe a), welcher diskrete Partikel eines Latex-Trägers umfasst, an welche ein Proteinmaterial kovalent gebunden ist, mit einem Aktivierungsmittel aktiviert.

Für die Zwecke der vorliegenden Erfindung geeignete Aktivierungsmittel sind in der Regel polyfunktionelle Verbindungen, welche zwei oder mehrere reaktive Gruppen aufweisen. Beispiele solcher Gruppen sind Azo-, Sulfonsäure-, durch Nitrogruppen aktivierte Fluorgruppen, Azid- und Imingruppen sowie reaktive Chlorgruppen, wie Chlorgruppen, welche an Ringe mit geeigneten Resonanzstrukturen gebunden sind. Diese reaktiven Gruppen sind fähig, mit den primären Aminogruppen, Sulfhydryl(mercapto)-Gruppen, Carboxyl- und Hydroxyl-Gruppen im Proteinmaterial zu reagieren, welche die Oberfläche der Trägerpartikel und des immunologisch aktiven Materials bilden.

Eine repräsentative Liste solcher Aktivierungsmittel umfasst: Bis-Diazobenzidin, Bis-Diazobenzidin-disulfonsäure, Tetraazo-p-phenylendiamin, Difluordinitrobenzol, Difluordinitrodiphenylsulfon, ein Carbodiimid der Formel I, Toluol-2,4-diisocyanat, Trichlor-s-triazin, Dichlor-s-triazin, N-t-Butyl-5-methyl-isoxazolium-p-chlorat, ein Dialdehyd, ein α,ß-ungesättigter Aldehyd, sowie Mischungen davon. Besonders geeignete Aktivierungsmittel sind Bis-Diazobenzidin-disulfonsäure und Toluol-2,4-diisocyanat.

Die Bedingungen bei der Aktivierung hängen vom spezifischen Aktivierungsmittel ab und können innerhalb weiter Grenzen variieren. Es ist indessen bevorzugt, bei einem pH von ungefähr 3 bis 9 und einer Temperatur von 0 bis 50°C zu arbeiten.

Das Aktivierungsmitel wird bevorzugt in grossem Ueberschuss verwendet. Nach der Rektion wird überschüssiges Aktivierungsmittel, z.B. durch Waschen in der Kälte, entfernt.

Nachdem das überschüssige Aktivierungsmittel durch Waschen entfernt wurde, wird der so aktivierte Latex

mit dem immunologisch aktiven Material zur Rektion gebracht. Obwohl der aktivierte Latex gelagert werden kann, ist es bevorzugt, die Reaktion mit dem immunologisch aktiven Material kurz nach der Aktivierung durchzuführen.

Das immunologische Material wird bevorzugt im Ueberschuss zugegeben und unter Bedingungen, welche die biologische Aktivität des immunologisch aktiven Materials nicht wesentlich beeinflussen. Der pH ist bevorzugt von 4 bis 9 und die Temperatur liegt zwischen 0 und 50°C.

Das Endprodukt wird vorzugsweise mehrmals mit Puffer gewaschen, und das überschüssige immunologisch aktive Material wird wiedergewonnen.

Die Menge an immunologisch aktivem Material, das an den neuen Latex gebunden ist, beträgt gewöhnlich etwa 0,01 bis 15,0 Gewichtsprozent. Jedes besondere immunologisch aktive Material wird jedoch in einer Menge verwendet, in der es bei einem Diagnosetest am erfolgreichsten eingesetzt wird. Daher wird jedes Material mit dem aktivierten Träger in einem Verhältnis kombiniert, das für die speziellen Erfordernisse geeignet ist. Die Erfindung umfasst daher die Verwendung einer Menge immunologisch aktiven Materials in Kombination mit dem aktivierten Latex, die ausreicht, ein diagnostisch wirksames Reagens zu liefern.

Ist das Produkt einmal gebildet, kann es in spezifischen Diagnosetests unter Anwendung immunologischer Prinzipien eingesetzt werden. Es kann in jeder passenden Konzentration in Abhängigkeit von dem speziellen Test verwendet werden, Konzentrationen von etwa 0,5 bis etwa 2,5 Gewichtsprozent sind jedoch geeignet, wobei 1,2 Gewichtsprozent bevorzugt werden. So kann beispielsweise das Produkt, das durch Kuppeln von Human-Myoglobin an den neuen aktivierten Latex entsteht, als Diagnosereagens zur Bestimmung von Myoglobinurie bei einem Patienten mit Verdacht auf Myokardinfarkt verwendet werden. Dies kann z.B. durch Aufbringen eines Tropfens Testurin auf einen sauberen Glasträger, Mischen mit einem Tropfen geeignet verdünnten Antihuman-Myoglobinserums und anschliessende Zugabe eines Tropfens des Myoglobin-Latex in wässriger Suspension erfolgen. Innerhalb einiger weniger Minuten sind die Testergebnisse zu beobachten, und zwar mit einer Genauigkeit von etwa 90-98 o/o.

Die Vorteile eines solchen Tests sind seine Einfachkeit, Schnelligkeit, Spezifität, Genauigkeit und das Fehlen falscher positiver Angaben.

Als weiteres Beispiel kann das Produkt, das durch Kuppeln von Schaf-Antihuman-IgG an den neuen aktivierten Latex entsteht, als Diagnosereagens zur Bestimmung von γ-Globin in Serum oder anderen Körperflüssigkeiten verwendet werden.

Das erfindungsgemässe immunologische Reagens kann zur Bestimmung immunologisch aktiver Materialien bei einem direkten oder indirekten (Hemmung) Agglutinationstest oder bei der in der deutschen Patentanmeldung P 2749956 offenbarten kinetisch-photometrischen Methode verwendet werden.

Bei dem direkten Test werden die Probe und die Latex-Teilchen, die mit dem Gegenreagens für das zu bestimmende immunologisch aktive Material überzogen sind, gemischt und die Agglutination visuell oder photometrisch beobachtet. Der Test ist positiv, wenn Agglutination eintritt.

Beim indirekten Test wird die Probe mit dem Gegenreagens (z.B. Antiserum) für das zu bestimmende Material und dann mit Latex-Teilchen, die mit dem gleichen Material überzogen sind, gemischt. Die Agglutination wird visuell oder photometrisch beobachtet. Der Test ist positiv, wenn keine Agglutination eintritt.

Die erfindungsgemässen immunologischen Reagenzien können für Handelszwecke bequem z.B. in einem Diagnosereagenssatz verpackt werden.

Für einen direkten Test enthält der Reagenssatz zur Bestimmung eines bekannten immunologisch aktiven Materials in einem Behälter ein wasserunlösliches immunologisches Reagens mit einem spezifischen Gewicht von etwa dem von Wasser, das diskrete Teilchen eines Latex-Trägers aufweist, an den ein Protein-Material kovalent gebunden ist, daran über eine kovalente Bindung ein immunologisches Gegenreagens für das zu bestimmende immunologisch aktive Material kondensiert.

Für einen indirekten Test enthält der Reagenssatz zur Bestimmung eines bekannten immunologisch aktiven Materials in einem ersten Behälter ein wasserunlösliches immunologisches Reagens mit einem spezifischen Gewicht von etwa dem von Wasser, das diskrete Teilchen eines Latex-Trägers aufweist, woran ein Proteinmaterial kovalent gebunden ist, daran über eine kovalente Bindung das zu bestimmende immunologisch aktive Material ankondensiert, und in einem zweiten Behälter ein immunologisches Gegenreagens für das zu bestimmende immunologisch aktive Material.

Die folgenden Beispiele veranschaulichen die Erfindung.

## Beispiel 1

Herstellung eines mit Rinderserum Albumin (bovine serum albumin; BSA) beschichteten Latex.

Carboxylierter Styrolbutadienlatex (Dow Cl 241) wird durch Waschen und Behandeln mit Ionenaustauscherharz Dow AG 50-X8; Bio Rad (Styroldivinylbenzol-polymerlatex mit Sulfonsäuregruppen am Kern) von Ammoniak befreit und in die Natriumform übergeführt.

18 ml dieses Latex Cl 241 in der $Na^+$-Form (Feststoffgehalt 78 mg/ml) werden auf pH 4,75 eingestellt. Zu der rasch gerührten Suspension wird eine Lösung von 30 mg CMC (1-Cyclohexyl-3-[2-morpholinyl-(4)-äthyl]-carbodiimid-metho-p-toluolsulfonat) in 6 ml Wasser zugegeben. Nach 3 Minuten werden 6 ml einer 1 o/o igen BSA-Lösung zugegeben. Nach der Zugabe des Proteins beträgt das pH der Suspension 5,7. Das Reaktionsgemisch wird während 24 Stunden bei Raumtemperatur gerührt. Darauf wird während 40 Minuten ei 44'000 x g zentrifugiert. Der Ueberstand wird verworfen und das Sediment in 40 ml Wasser suspendiert, mit einem Mixer homogenisiert und wieder zentrifugiert. Das Waschen wird viermal mit 0,1 M Boratpuffer vom pH 8,5 wiederholt, worauf zweimal mit destilliertem Wasser gewaschen wird. Der Latex wird schliesslich in 60 ml Wasser aufgenommen und durch Zugabe von 120 µl 20 o/oiger Natriumazidlösung stabilisiert.

## Beispiel 2

Herstellung eines mit Humanimmunglobulin G (IgG) beschichteten Latex.

6 ml eines gemäss Beispiel 1 vorbehandelten Latex Cl 241 (79,5 mg/ml) wird auf pH 4,75 gestellt und durch

Zugabe von 2 ml einer CMC-Lösung enthaltend 8 mg CMC aktiviert. Nach 3 Minuten wird die Latexsuspension in 8 ml einer rasch gerührten Lösung von Human IgG in Boratpuffer gegossen. 120 mg kommerziell erhältliches IgG werden in 12 ml 0,015 M Boratpuffer vom pH 8,5 suspendiert, während 1 Stunde gerührt, worauf das unlösliche Material durch Zentrifugation entfernt wird. Die optische Dichte dieser Lösung bei 280 nm ist 6,44 was einer Proteinkonzentration von ungefähr 5,1 mg/ml entspricht. Das Reaktionsgemisch wird bei Raumtemperatur während 24 Stunden gerührt und dann während 30 Minuten bei 45'000 x g zentrifugiert. (Der optischen Dichte des Ueberstandes die bei 280 nm abgelesen wird kann man entnehmen, dass praktisch alles IgG an den Latex gebunden wurde. Um unsedimentierte Latexpartikel vom Ueberstand zu entfernen wurde dieser durch eine Nucleopore-Membran mit einer effektiven Porengrösse von 0,1 μ filtriert, bevor die optische Dichte gemessen wurde). Der Latex wird viermal mit ungefähr 40 ml 0,1 M Boratpuffer vom pH 8,5 und dann zweimal mit destilliertem Wasser gewaschen. Es wird schliesslich in 20 ml Wasser aufgenommen und durch Zugabe von 40 μl 20 o/oiger Natriumazidlösung stabilisiert.

## Beispiel 3

Herstellung von Bis-Diazobenzidin-2,2'-disulfonsäure (BDBDS).

0,11 g (1,0 mMol) wasserfreies Natriumcarbonat werden in 3 ml destilliertem Wasser gelöst. Zu dieser Lösung werden 0,34 g /1,0 mMol) Benzidin-2,2'-disulfonsäure (BDS) gegeben. Nachdem die Lösung klar geworden ist, werden 0,142 g (2,05 mMol) Natriumnitrit zugegeben. Das Reaktionsgefäss wird in ein Eisbad getaucht, worauf dem Reaktionsgemisch 0,6 ml 30 o/oige Salzsäure (4,9 mMol) unter raschem Rühren zugegeben werden. Nach 5

Minuten werden 14,6 mg (0,15 mMol) Sulfamidsäure zugegeben, worauf die Suspension im Eisbad gehalten und gerührt wird. Minuten später fällt kristallines BDBDS von der gelben Lösung aus. Nach Stehenlassen der Suspension bei 0°C verwandelt sich der BDBDS-Niederschlag in feine Nadeln.

Zur Aktivation des mit Protein beschichteten Latex wird die erhaltene BDBDS-Suspension zentrifugiert, wobei entweder der Ueberstand oder eine Suspension des festen BDBDS in 0,01 n Salzsäure verwendet wird.

## Beispiel 4

Aktivierung von mit Rinderserum Albumin (BSA) beschichtetem Latex mit Bis-Diazobenzidin-2,2'-disulfonsäure (BDBDS).

a) Zu 5 ml von mit BSA beschichtetem Latex werden bei 0°C 2 ml Ueberstand gemäss Beispiel 3 enthaltend BDBDS gegeben. Nach dieser Reaktion und durch die Zugabe von 1 ml 1 M Natriumboratpuffer vom pH 8,1 und Tropfen von 1 n Natriumhydroxidlösung steigt der pH von 2 auf 8,5. Das Reaktionsgemisch wird während 10 Minuten gerührt und dann bei 0°C während 30 Minuten bei 45'000 x g zentrifugiert. Der Ueberstand wird dekantiert und für die Farbbestimmung von BDBDS aufbewahrt. Das gelbe Sediment wird in ungefähr 12 ml eiskaltem 0,1 M Boratpuffer vom pH 8,5 suspendiert. Die Latexsuspension wird während 30 Minuten erneut zentrifugiert. Das Sediment wird in 0,5 ml eiskaltem Boratpuffer suspendiert.

b) Zu 5 ml einer rasch gerührten Suspension von mit BSA beschichtetem Latex werden bei 0°C 0,5 ml einer Suspension von BDBDS in 0,01 n Salzsäure (hergestellt nach Beispiel 3) zugegeben. (Nach Zentrifugation des

BDBDS-Niederschlages wurde das kristalline BDBDS in ungefähr 15 ml 0,01 n Salzsäure suspendiert). Durch Zugabe von 1 ml 1 M Boratpuffer vom pH 8,1 steigt der pH des Reaktionsgemisches auf 8,5. Nach 15 Minuten wird das Reaktionsgemisch mit ungefähr 12 ml eiskaltem destilliertem Wasser verdünnt. Man zentrifugiert während 30 Minuten bei 45'000 x g und 2°C. Der Ueberstand wird dekantiert und für die Farbbestimmung des BDBDS mit α-Naphtol aufbewahrt. Der aktivierte Latex wird mit ungefähr 12 ml eiskaltem 0,1 M Boratpuffer vom pH 8,5 gewaschen, rezentrifugiert und in 0,5 ml kaltem Boratpuffer suspendiert.

## Beispiel 5

Aktivierung von mit Immunoglobulin G (IgG) beschichtetem Latex mit Bis-Diazobenzidin-2,2'-disulfonsäure (BDBDS).

Zu einer gerührten Suspension von 5 ml eines mit menschlichem IgG beschichtetem Latex werden bei 0°C 1,5 ml vom Ueberstand (hergestellt gemäss Beispiel 3) enthaltend BDBDS gegeben. Nach der Reaktion und nach Zugabe von 1 ml 1 M Natriumboratpuffer vom pH 8,1 und Tropfen von 1 n Natriumhydroxidlösung steigt das pH auf 8,5. Das Reaktionsgemisch, welches einige Gasblasen entwickelt (offensichtlich von freigesetztem Stickstoff) wird während 10-15 Minuten gerührt und dann während 30 Minuten bei 45'000 x g und 2°C zentrifugiert. Der Ueberstand wird dekantiert und das Sediment mit ungefähr 12 ml eiskaltem 0,1 M Boratpuffer vom pH 8,5 gewaschen und suspendiert. Der Latex wird während 25 Minuten bei 2°C zentrifugiert. Nach Abdekantieren wird das Sediment in 0,2 ml eiskaltem 0,1 M Boratpuffer suspendiert.

## Beispiel 6

Aktivierung eines mit BSA beschichtetem Latex mit Toluol-2,4-diisocyanat (TDIC).

Zu einer gerührten Suspension von 5 ml eines mit BSA beschichtetem Latex werden 0,5 ml 1 M Natriumboratpuffer vom pH 8,1, 1 ml Dioxan und 0,2 ml einer Lösung von 20 µl TDIC in 4 ml Dioxan gegeben. Das pH des Reaktionsgemisches ist 9,25. Nach 45 Minuten wird mit Wasser auf 12 ml verdünnt und dann während 30 Minuten bei 45'000 x g zentrifugiert. Das Sediment wird wieder in 0,1 M Boratpuffer vom pH 8,5 suspendiert. Der Latex wird mit ungefähr 12 ml dieses Puffers gewaschen. Nach Zentrifugation wird das Sediment direkt in der Trägerproteinlösung suspendiert (vgl. Beispiel 13).

## Beispiel 7

Aktivierung eines mit IgG beschichteten Latex mit TDIC. Das Verfahren gemäss Beispiel 6 wird wiederholt, wobei an Stelle von BSA IgG verwendet wird.

## Beispiel 8

Bindung von Human-Plazenta-Lactogen (HPL) an einen mit Rinderserum Albumin (BSA) beschichteten und mit Bis-Diazobenzidin-2,2'-disulfonsäure (BDBDS) aktivierten Latex.

Der gemäss Beispiel 4a) erhaltene aktivierte, gewaschene Rückstand von 5 ml mit BSA beschichtetem Latex wird in 1 ml einer eiskalten 1 o/oigen HPL-Lösung in 0,1 M Natriumboratpuffer vom pH 8,5 suspendiert. Hierauf lässt man unter Rühren während 48 Stunden im Dunkeln bei 4°C weiterreagieren. Nach dieser Zeit wird der Latex während 30 Minuten bei 2°C und 45'000 x g zentrifugiert.

Der Ueberstand wird abgesaugt und zur Wiederverwendung von überschüssigem HPL aufbewahrt. Das Sediment wird in ungefähr 12 ml 0,1 M Glycinpuffer vom pH 8,2 suspendiert. Man zentrifugiert wieder und wäscht dreimal mit derselben Menge Puffer. Schliesslich wird das Sediment in 5 ml Glycinpuffer (stabilisiert mit 0,04% Natriumazid) aufgenommen.

20 µl dieses Latex, gemischt mit 60 µl phosphatgepufferter Kochsalzlösung (phosphate bufferd saline; PBS) und 20 µl Kaninchen anti HPL-Antiserum (1:30 in PBS) zeigt nach 25 Sekunden sichtbare Agglutination. Falls 0,5 µg HPL mit dem Antiserum vorgemischt werden, kann man nach 4,5 Minuten keine Agglutination beobachten.

## Beispiel 9

Kupplung von Human-Plazenta-Lactogen (HPL) an einen mit Humanimmunglobulin (IgG) beschichteten und mit Bis-Diazobenzidin-2,2'-disulfonsäure (BDBDS) aktivierten Latex.

Der gemäss Beispiel 5 erhaltene, aktivierte und gewaschene Rückstand von 5 ml mit Human IgG beschichtete Latex wird in 0,2 ml eiskaltem 0,1 M Boratpuffer vom pH 8,5 suspendiert. 1 ml einer 1 o/oigen wässrigen Lösung von HPL wird zugegeben, worauf man die Kupplungsreaktion in der Dunkelheit bei 4°C während 72 Stunden unter Rühren weiterlaufen lässt. Der Latex wird dann zentrifugiert, und der Ueberstand wird abpipettiert und für Wiederverwendung von überschüssigem HPL aufbewahrt. Das Sediment wird in ungefähr 12 ml Glycinpuffer suspendiert. Es wird rezentrifugiert und dreimal in Glycinpuffer suspendiert. Das Sediment wird schliesslich in 5 ml dieses Puffers suspendiert.

20 µl dieses Latex gemischt mit 60 µl PBS und 20 µl Kaninchen anti HPL-Antiserum (1:80 in PBS) zeigt nach 15 Sekunden sichtbare Agglutination. Falls 0,5 µg HPL dem Antiserum beigemischt werden, kann nach 5 Minuten keine Agglutination beobachtet werden.

Beispiel 10

Kupplung von Humanmyoglobin an einen mit Rinderserum Albumin (BSA) beschichteten und mit Bis-Diazobenzidin-2,2'-disulfonsäure (BDBDS) aktivierten Latex.

5 ml eines mit BSA beschichteten Latex werden mit 1 ml BDBDS Suspension in 0,01 n Salzsäure aktiviert, worauf der Latex wie in Beispiel 4b) behandelt wird. Das Sediment des gewaschenen, aktivierten Latex wird in 1 ml 0,1 M Boratpuffer vom pH 8,5 enthaltend 20 mg Humanmyoglobin suspendiert. Die Kupplungsreaktion wird während 60 Stunden unter Rühren bei 4°C in der Dunkelheit weitergeführt. Der Latex wird dann zentrifugiert, worauf der Ueberstand abpipettiert und zur Wiederverwendung von überschüssigem Myoglobin aufbewahrt wird. Das Sediment wird in 12 ml Glycinpuffer suspendiert und gewaschen. Das Waschen des Latex wird dreimal mit demselben Puffer wiederholt. Der Latex wird schliesslich in 5 ml Glycinpuffer (stabilisiert mit 0,04 o/o Natriumazid) aufgenommen.

20 µl dieses Latex, gemischt mit 40 µl Veronalacetatpuffer vom pH 8,2 (je 48 mMol Natriumacetat und Natrium-diäthyl-barbiturat [pH mit Eisessig eingestellt], 20 µl normaler menschlicher Urin und 20 µl Kaninchenantihumanmyoglobin-Antiserum (verdünnt 1:20 in Veronalacetat) zeigt nach 40 Sekunden sichtbare Agglutination. Mit Urin enthaltend 5 µg/ml Myoglobin kann während 9 Minuten keine Agglutination beobachtet werden.

## Beispiel 11

Kupplung von Schaf anti human IgG Antikörpern auf einen mit Rinderserum Albumin (BSA) beschichteten und mit Bis-Diazobenzidin-2,2'-disulfonsäure (BDBDS) aktivierten Latex.

5 ml eines mit BSA beschichteten Latex wird mit 0,5 ml einer Suspension von BDBDS in 0,01 n Salzsäure aktiviert, worauf der Latex wie in Beispiel 4b) behandelt wird. Das Sediment des gewaschenen, aktivierten Latex wird in 0,5 ml eiskaltem 0,1 M Boratpuffer vom pH 8,5 suspendiert, worauf 1 ml Antikörperlösung in 0,1 ml Natriumbicarbonat vom pH 8,5 zugegeben werden. (Die Antikörperlösung wurde durch Affinitätschromatographie von Antiserum an human IgG-Sepharose erhalten). Die Kupplungsreaktion wird während 72 Stunden unter Rühren bei 4°C in der Kälte weitergeführt. Der Latex wird dann zentrifugiert, worauf der Ueberstand zur Wiederverwendung von überschüssigem Antikörper aufbewahrt wird. Von der optischen Dichte des Ueberstandes kann man berechnen, dass ungefähr 2,1 mg Antikörper gebunden wurde. Der Latex wird viermal mit Glycinpuffer gewaschen und schliesslich in 5 ml dieses Puffers (stabilisiert mit 0,04 o/o Natriumazid) aufgenommen.

20 µl dieses Latex vermischt mit 80 µl veronalgepufferter Kochsalzlösung (145 mMol Natriumchlorid, 3,13 mMol Diäthylbarbitursäure, 1,8 mMol Natriumdiäthylbarbiturat) enthaltend 0,2 µg Human IgG ergab eine spezifische Agglutination, welche nach 25 Sekunden sichtbar war.

Beispiel 12

Kupplung von Streptolysin O an einen mit Rinderserum Albumin (BSA) beschichteten und mit Bis-Diazobenzidin-2,2'-disulfonsäure (BDBDS) aktivierten Latex.

5 ml eines mit BSA beschichteten Latex werden mit 2 ml Ueberstand enthaltend BDBDS /vgl. Beispiel 4a) aktiviert. Der aktivierte, gewaschene Rückstand wird in 1 ml einer eiskalten Streptolysin O-Zubereitung in phosphatgepufferter Kochsalzlösung vom pH 8,5 suspendiert. Diese Streptolysin O-Zubereitung zeigt einen Titer von 1160 in der hämolytischen Bestimmung (bei 50 o/o Lysis der Zellen). Nach einer Reaktionszeit von 48 Stunden wird der Latex zentrifugiert und der Ueberstand wird in der hämolytischen Bestimmung getestet. Er zeigt nun einen Titer von 6, was bedeutet, dass das meiste Streptolysin reagiert hat und an den Latex gebunden wurde. Der Latex wird wieder suspendiert und viermal mit 0,1 M Glycinpuffer vom pH 8,2 gewaschen und schliesslich in 5 ml dieses Puffers (stabilisiert mit 0,04 o/o Natriumazid) aufgenommen.

25 µl dieses Latex vermischt mit 50 µl PBS und 25 µl Kaninchenantistreptolysin-Antiserum (bereits verdünnt) zeigt nach 35 Sekunden deutliche Agglutination.

Beispiel 13

Kupplung von Human-Plazenta-Lactogen (HPL) an einen mit Rinderserum Albumin (BSA) beschichteten und mit TDIC aktivierten Latex.

Der aktivierte, gewaschene Rückstand von 5 ml mit BSA beschichtetem Latex (vgl. Beispiel 6) wird in 1 ml einer 1 o/oigen HPL-Lösung in Wasser suspendiert. Die Kupplungsreaktion wird während 96 Stunden unter Rühren

bei $4^oC$ weitergeführt. Der Latex wird dann zentrifugiert, worauf überschüssiges HPL vom Ueberstand zurückgewonnen wird. Der Latex wird wieder suspendiert und viermal mit Glycinpuffer (ungefähr jeweils 12 ml) gewaschen und schliesslich in 5 ml dieses Puffers (stabilisiert mi 0,04 o/oigem Natriumazid) aufgenommen.

20 µl dieses Latex, vermischt mit 60 µl PBS und 20 µl Kaninchen anti HPL-Antiserum (1:40 PBS) zeigt nach 10 Sekunden sichtbare Agglutination. Falls 1 µg HPL mit dem Antiserum vorgemischt wird, erfolgt Agglutination erst nach 1 1/2 Minuten und führt nach 5 Minuten zu einem feinen Niederschlag.

## Beispiel 14

Kupplung von Human-Plazenta-Lactogen (HPL) an einen mit Humanimmunoglobulin G (IgG) beschichteten und mit TDIC aktivierten Latex.

Der aktivierte, gewaschene Rückstand von 2,5 ml eines mit Human-IgG beschichteten Latex (vgl. Beispiel 7) wird in 0,5 ml einer 1 o/oigen HPL-Lösung in Wasser suspendiert. Dieser wird in gleicher Weise behandelt wie in Beispiel 13 und schliesslich in 2,5 ml Glycinpuffer (stabilisiert mit 0,4 o/oigem Natriumazid) aufgenommen.

20 µl dieses Latex, vermischt mit 60 µl PBS und 20 µl Kaninchen-HPL-Antiserum (1:40 in PBS) zeigt nach 15 Sekunden sichtbare Agglutination. Falls 0,5 µg HPL mit dem Antiserum vorgemischt werden, kann während 6 Minuten keine sichtbare Agglutination beobachtet werden.

## Patentansprüche

1. Wasser-unlösliches, immunologisches Reagens mit einem spezifischen Gewicht von etwa dem von Wasser, mit diskreten Teilchen eines Latex-Trägers, an den ein Proteinmaterial kovalent gebunden ist, woran über eine kovalente Bindung ein immunologisch aktives Material kondensiert ist.

2. Reagens nach Anspruch 1, dadurch gekennzeichnet, dass die Teilchen des Latex-Trägers eine Grösse im Bereich von etwa 0,01 bis etwas 0,9 µm aufweisen.

3. Reagens nach Anspruch 1 oder 2, dadurch gekennzeichnet, dass der Latex-Träger ein carboxylierter Latex ist.

4. Reagens nach Anspruch 3, dadurch gekennzeichnet, dass der carboxylierte Latex ein carboxyliertes Styrol-Butadien-Copolymerisat ist.

5. Reagens nach einem der Ansprüche 1 bis 4, dadurch gekennzeichnet, dass das Proteinmaterial Rinderserumalbumin ist.

6. Reagens nach einem der Ansprüche 1 bis 4, dadurch gekennzeichnet, dass das Proteinmaterial IgG ist.

7. Reagens nach einem der Ansprüche 1 bis 6, dadurch gekennzeichnet, dass das Verhältnis vom Träger-Latex zum Proteinmaterial zwischen 2 und 50 liegt.

8. Reagens nach Anspruch 7, dadurch gekennzeichnet, dass das Verhältnis vom Träger-Latex zum Proteinmaterial zwischen 10 und 30 liegt.

9. Reagens nach einem der Ansprüche 1 bis 8, dadurch gekennzeichnet, dass das immunologisch aktive Material Mycglobin ist.

10. Reagens nach einem der Ansprüche 1 bis 8, dadurch gekennzeichnet, dass das immunologisch aktive Material Human-Plazenta-Lactogen ist.

11. Reagens nach einem der Ansprüche 1 bis 8, dadurch gekennzeichnet, dass das immunologisch aktive Material ein Antikörper gegen IgG ist.

12. Reagens nach einem der Ansprüche 1 bis 8, dadurch gekennzeichnet, dass das immunologisch aktive Material Streptolysin O ist.

13. Aktivierter Latex, dadurch gekennzeichnet, dass er durch Behandeln diskreter Latex-Teilchen, auf welche ein Proteinmaterial kovalent gebunden ist, mit einem Aktivierungsmittel erhalten wird.

14. Latex nach Anspruch 13, dadurch gekennzeichnet, dass die Teilchen des Latex-Trägers eine Grösse im Bereich von etwa 0,01 bis etwa 0,9 µm haben.

15. Latex nach Anspruch 13 oder 14, dadurch gekennzeichnet, dass das spezifische Gewicht der Teilchen des Latex-Trägers etwa 1 ist.

16. Latex nach einem der Ansprüche 13 bis 15, dadurch gekennzeichnet, dass der Latex-Träger ein carboxylierter Latex ist.

17. Latex nach Anspruch 16, dadurch gekennzeichnet, dass der carboxylierte Latex ein carboxyliertes Styrol-Butadien-Copolymerisat ist.

18. Latex nach einem der Ansprüche 13 bis 17, dadurch gekennzeichnet, dass das Proteinmaterial Rinderserumalbumin ist.

19. Latex nach einem der Ansprüche 13 bis 17, dadurch gekennzeichnet, dass das Proteinmaterial Immunglobulin G ist.

20. Latex nach einem der Ansprüche 13 bis 19, dadurch gekennzeichnet, dass das Verhältnis vom Träger-Latex zum Proteinmateial zwischen 2 und 50 liegt.

21. Latex gemäss Anspruch 20, dadurch gekennzeichnet, dass das Verhältnis vom Träger-Latex zum Proteinmaterial zwischen 10 und 30 liegt.

22. Verfahren zur Herstellung eines aktivierten Latex gemäss einem der Ansprüche 13 bis 21, dadurch gekennzeichnet, dass diskrete Partikel eines Latex-Trägers, an welche ein Proteinmaterial kovalent gebunden ist, mit einem Aktivierungsmittel behandelt werden.

23. Verfahren nach Anspruch 22, dadurch gekennzeichnet, dass das Aktivierungsmittel Bis-Diazo-benzidin-2,2'-disulfonsäure ist.

24. Verfahren nach Anspruch 22, dadurch gekennzeichnet, dass das Aktivierungsmittel Toluol-2,4-diisocyanat ist.

25. Verfahren zur Herstellung eines Reagens gemäss einem der Ansprüche 1 bis 12, dadurch gekennzeichnet, dass ein immunologisch aktives Material in Gegenwart eines Aktivierungsmittels mit einem aktivierten Latex behandelt wird, welcher durch Behandeln diskreter Teilchen eines Latex-Trägers, an welche ein Proteinmaterial kovalent gebunden ist, hergestellt wurde.

26. Verfahren zum Bestimmen eines immunologisch aktiven Materials in einer Probe, bei dem die Probe mit einem wasser-unlöslichen immunologischen Reagens mit einem spezifischen Gewicht von etwa dem von Wasser, mit diskreten Teilchen eines Latex-Trägers, an dem ein Proteinmaterial kovalent gebunden ist, daran über eine kovalente Bindung ein immunologisches Gegenreagens für das zu bestimmende immunologisch aktive Material ankondensiert, in Berührung gebracht und das Eintreten einer Agglutination beobachtet wird.

27. Verfahren nach Anspruch 26, dadurch gekennzeichnet, dass als zu bestimmendes immunologisch aktives Material γ-Globulin und als immunologisches Gegenreagens Schaf Antihuman IgG verwendet wird.

28. Vefahren zum Bestimmen eines immunologisch aktiven Materials in einer Probe, bei dem die Probe mit einem immunologischen Gegenreagens für das zu bestimmende immunologisch aktive Material und mit einem wasser-unlöslichen immunologischen Reagens mit einem spezifischen Gewicht von etwa dem von Wasser, mit diskreten Teilchen eines Latex-Trägers, an den ein Proteinmaterial kovalent gebunden ist, daran über eine kovalente Bindung das zu bestimmende immunologisch aktive Material ankondensiert, in Berührung gebracht und das Eintreten einer Agglutination beobachtet wird.

29. Verfahren nach Anspruch 26, dadurch gekennzeichnet, dass als zu bestimmendes immunologisch aktives Material Myoglobin und als immunologisches Gegenreagens Myoglobin Antiserum verwendet wird.

30. Reagenssatz für die Bestimmung eines bekannten immunologisch aktiven Materials, enthaltend in einem Behälter ein wasser-unlösliches immunologisches Reagens mit einem spezifischen Gewicht von etwa dem von Wasser, das diskrete Teilchen eines Latex-Trägers aufweist, an den ein Proteinmaterial gebunden ist, daran über eine kovalente Bindung ein immunologisches Gegenreagens für das zu bestimmende immunologisch aktive Material ankondensiert.

31. Reagenssatz nach Anspruch 30, dadurch gekennzeichnet, dass das zu bestimmende immunologisch aktive Material γ-Globulin und dessen immunologischen Gegenreagens Schaf Antihuman IgG ist.

32. Reagenssatz zur Bestimmung eines bekannten immunologisch aktiven Materials, enthaltend in einem ersten Behälter ein wasser-unlösliches immunologisches Reagens mit einem spezifischen Gewicht von etwa dem von Wasser, das diskrete Teilchen eines Latex-Trägers aufweist, an den ein Proteinmaterial kovalent gebunden ist, daran über eine kovalente Bindung das zu bestimmende immunologisch aktive Material ankondensiert, und in einem zweiten Behälter ein immunologisches Gegenreagens für das zu bestimmende immunologisch aktive Material.

33. Reagenssatz nach Anspruch 32, dadurch gekennzeichnet, dass das zu bestimmende immunologisch aktive Material Myoglobin und das immunologische Gegenreagens Myoglobin Antiserum ist.

34. Verwendung eines Reagens gemäss einem der Ansprüche 1 bis 12 bei immunologischen Bestimmungen.

35. Verwendung eines aktivierten Latex gemäss einem der Ansprüche 13 bis 21 als Träger bei immunologischen Bestimmungen.

********

Europäisches Patentamt

# EUROPÄISCHER RECHERCHENBERICHT

Nummer der Anmeldung

EP 79 102 770.9



| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| P,A | EP - A1 - 0 001 224 (F. HOFFMANN-LA ROCHE)<br>* ganzes Dokument *<br>-- | | |
| P,A | EP - A2 - 0 001 223 (F. HOFFMANN-LA ROCHE)<br>* ganzes Dokument *<br>---- | | |
| | | | RECHERCHIERTE SACHGEBIETE (Int. Cl.3) |

0008682

Europäisches Patentamt

# EUROPÄISCHER RECHERCHENBERICHT

Nummer der Anmeldung

EP 79 102 770.9

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| | US - A - 3 551 555 (ORGANON)<br>* ganzes Dokument *<br>& DE - A - 1 598 859<br>-- | 1-6,<br>25 | G 01 N 33/54<br>C 07 G 7/00<br>C 08 F 8/00 |
| | GB - A - 1 252 770 (MILES LABORATORIES)<br>* Ansprüche 1 bis 6, 9 *<br>-- | 1,<br>22-25 | |
| | GB - A - 1 252 769 (MILES LABORATORIES)<br>* Ansprüche 2, 13, Seite 4, Zeilen 27 bis 40 *<br>-- | 1,8 | RECHERCHIERTE SACHGEBIETE (Int. Cl.3) |
| | CH - A5 - 591 694 (F. HOFFMANN-LA ROCHE)<br>* Patentanspruch 1, Unteransprüche 1 bis 8, Patentanspruch 2, Unteransprüche 9, 10 *<br>-- | 26-28,<br>30-32 | C 07 G 7/00<br>C 08 F 8/00<br>G 01 N 33/54 |
| A | AT - B - 322 733 (F. HOFFMANN-LA ROCHE)<br>* ganzes Dokument *<br>-- | | |
| A | US - A - 4 045 384 (DOW CHEMICAL)<br>* ganzes Dokument *<br>-- | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| P,A | DE - A1 - 2 812 845 (ORTHO DIAGNOSTICS)<br>* ganzes Dokument *<br>-- | | |
| P,A | EP - A1 - 0 000 772 (F. HOFFMANN-LA ROCHE)<br>* ganzes Dokument * ./..<br>-- | | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 12-11-1979 | SCHWARTZ |